# EUROPEAN PATENT APPLICATION

(11) **EP 3 978 486 A1**
(43) Date of publication of application: **06.04.2022**
(21) Application number: 20814441.0
(22) Date of filing: 22.05.2020
(51) Int. Cl.: C07D 401/12, A61K 31/506, A61K 31/513, A61P 35/00

(54) **CRYSTAL FORM OF C-MET/AXL INHIBITOR**

(30) Priority: 24.05.2019 CN 201910439448
(71) Applicant: Medshine Discovery Inc., Nanjing, Jiangsu 210032 (CN)
(72) Inventor: LI, Gang, Shanghai 200131 (CN); WANG, Kun, Shanghai 200131 (CN); HU, Lihong, Shanghai 200131 (CN); DING, Charles Z., Shanghai 200131 (CN)
(74) Representative: Henderson, Helen Lee
(86) International application number: PCT/CN2020/091889
(87) International publication number: WO 2020/238802

(57) **Abstract**

A crystal form and a salt form of a uracil compound acting as a c-MET/AXL inhibitor and a preparation method therefor, specifically relating to the crystal form and the salt form of the compound shown in formula (I), and also comprising an application of the crystal form and the salt form in the preparation of drugs for the treatment of tumours.

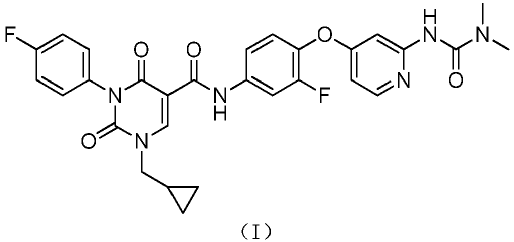

## Description

The present application claims the following priority of:
CN201910439448.6, filing date: May 24, 2019.

### Technical Field

The present disclosure relates to a crystal form and a salt form of a uracil compound of a c-MET/AXL inhibitor, and a preparation method therefor, and a use of the crystal form and the salt form in the manufacture of a medicament for treating a tumor.

### Background

The c-Met encoded by proto-oncogene Met is a receptor tyrosine kinase with high affinity belonging to RON subgroup. It is the only known receptor for scattering factor or hepatocyte growth factor (HGF). C-Met protein is a heterodimer containing 50 kD α subunit and 145 kD β subunit connected by disulfide bonds, and is divided into extracellular domain and intracellular domain. The extracellular domain contains three domains with different functions: the N-terminal ligand-binding domain (SEMA region) covering the entire α-chain and part of the β-chain, the cysteine-rich domain with four conserved disulfide bonds, and the immunoglobulin-like structural domain. The intracellular domain is also composed of three regulatory regions: the membrane-proximal domain with Tyr1003 phosphorylation sites, the tyrosine kinase catalytic domain with Tyr1234 and Tyr1235 phosphorylation sites, and the C-terminal multifunctional binding region with Tyr1349 and Tyr1356 binding to tyrosine.

HGF induces phosphorylation of c-Met by binding to its extracellular domain, and recruits a variety of interstitial factors such as GAB1 (growth factor receptor binding protein-1) and GAB2 (growth factor receptor binding protein-2) in the C-terminal multifunctional domain, further attracting molecules such as SHP2, PI3K etc., to bind here, hence activating RAS/MAPK, PI3K/AKT, JAK/STAT pathways etc., thereby regulating the growth, migration, proliferation and survival of cells. Abnormal action of the c-Met pathway would lead to tumorigenesis and metastasis, and abnormal high expression of c-Met has been found in various human malignancies such as bladder cancer, gastric cancer, lung cancer and breast cancer. In addition, c-Met is also associated with drug resistance to multiple kinase inhibitors in tumors.

The crosstalk between c-Met and various membrane receptors (crosstalk) constitutes a complex network system. The crosstalk between c-Met and adhesion receptor CD44 amplifies the response of signal peptide; the crosstalk between c-Met and the brain protein receptor activates c-Met level of independent ligand HGF, and then enhances the invasion effect; the crosstalk between c-Met and the pro-apoptotic receptor FAS accelerates apoptosis; the crosstalk between c-Met and various receptor tyrosine kinases such as EGFR, VEGFR regulates the activation between each other, thus affecting the angiogenesis process. The crosstalk between c-Met and these membrane receptors promotes tumorigenesis, metastasis and induces drug resistance.

AXL is a transmembrane protein. The extracellular domain includes two immunoglobulin-like domains and two fibronectin-like domains. The ligand binding domain is an immunoglobulin-like domain. AXL, Tyro3 and Mer belong to the TAM receptor tyrosine kinase family, and all of them use the protein molecule encoded by growth arrest specific gene 6 (Gas6) and human plasma anticoagulant protein S as ligands. When AXL binds to Gas6, the conformation of AXL changes to form a dimer. The tyrosine residues in the inner membrane are phosphorylated, activates the tyrosine protein kinase activity of AXL itself, further phosphorylates downstream proteins and plays a role in signal transduction. AXL activation can cause GRB2 activation, which in turn affects tumor cell proliferation through the RAS-RAF-MEK-ERK signaling pathway, and can also phosphorylate PI3K, which in turn activates AKT and enhances tumor cell survival. In addition, AXL can directly activate SRC or promote tumor cell migration and invasion by interacting with EGFR, VEGFR and MET, leading to metastatic progression. The high expression of AXL protein is associated with the deterioration of breast cancer, lung cancer, and acute myelogenous leukemia. Studies have shown that AXL signal activation is one of the main mechanisms of epithelial-mesenchymal transition (EMT) in tumor cells, as well as one of the main mechanisms for cancer cells to develop resistance to targeted drugs and chemotherapeutic drugs.

At present, there are many anti-tumor drugs on the market, such as alkylating drugs, antimetabolites, anti-tumor antibiotics, immunomodulators, etc., but most of them are not tolerated by patients due to their high toxicity. With the deepening research of tumor molecular biology, the molecular mechanism of tumor occurrence and development has become more and more clear. Molecular targeted therapy of various malignant tumors has received extensive attention and great attention. Molecular targeted drugs are highly selective, broad-spectrum and effective, and their safety is better than cytotoxic chemotherapeutic drugs, which is a new direction in the development of tumor therapy.

### Content of the present invention

The present disclosure provides a crystal form A of a compound represented by formula (I), the X-ray powder diffraction pattern thereof has characteristic diffraction peaks at the following 2θ angles: 9.37° ± 0.20°, 17.17° ± 0.20°, and 18.89° ± 0.20°.

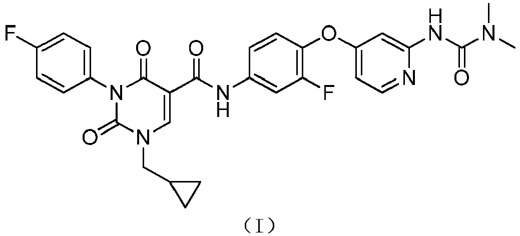

In some embodiments of the present disclosure, the X-ray powder diffraction pattern of the crystal form A has characteristic diffraction peaks at the following 2θ angles: 9.37°±0.20°, 10.37±0.20°, 12.92±0.20°, 17.17±0.20°, 18.89±0.20°, 19.82±0.20°, 22.09±0.20° and 24.48±0.20°.

In some embodiments of the present disclosure, the X-ray powder diffraction pattern of the crystal form A has characteristic diffraction peaks at the following 2θ angles: 8.10°, 9.37°, 10.37°, 10.92°, 12.92°, 14.11°, 14.67°, 15.21°, 15.85°, 16.21°, 16.66°, 17.17°, 17.64°, 18.89°, 19.18°, 19.82°, 20.74°, 21.30°, 22.09°, 22.91°, 23.90°, 24.48°, 25.56°, 25.92°, 26.29°, 27.04°, 27.39°, 28.32°, 29.27°, 29.86°, 30.57°, 31.34°, 32.16°, 32.62°, 33.27°, 33.79°, 34.45°, 34.75°, 36.80° and 39.33°.

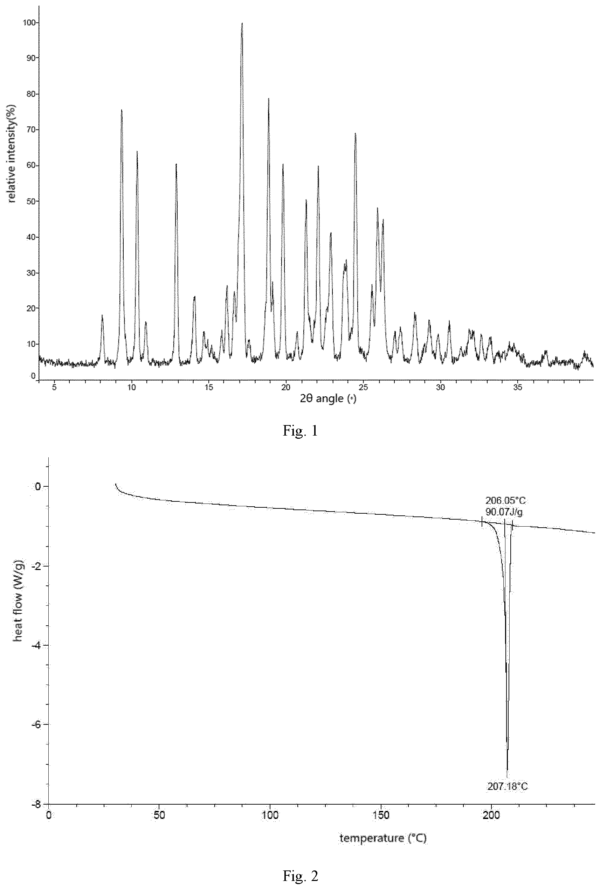

In some embodiments of the present disclosure, the X-ray powder diffraction pattern of the crystal form A is as shown in Fig.1.

In some embodiments of the present disclosure, the analytical data of the XRPD pattern of the crystal form A is as shown in Table 1.

**Table 1: Analytical data of the XRPD pattern of the crystal form A of the compound represented by formula (I)**

| **No.** | **2θ Angle (°)** | **d-Spacing (Å)** | **Relative intensity (%)** | **No.** | **2θ Angle (°)** | **d-Spacing (Å)** | **Relative intensity (%)** |
|---|---|---|---|---|---|---|---|
| 1 | 8.104 | 10.9004 | 8.0 | 21 | 23.901 | 3.7200 | 41.0 |
| 2 | 9.366 | 9.4346 | 51.6 | 22 | 24.475 | 3.6340 | 52.1 |
| 3 | 10.372 | 8.5221 | 39.5 | 23 | 25.559 | 3.4823 | 19.0 |
| 4 | 10.924 | 8.0924 | 9.5 | 24 | 25.915 | 3.4352 | 49.6 |
| 5 | 12.915 | 6.8490 | 34.7 | 25 | 26.286 | 3.3876 | 48.0 |
| 6 | 14.114 | 6.2695 | 12.1 | 26 | 27.039 | 3.2950 | 4.2 |
| 7 | 14.674 | 6.0315 | 13.7 | 27 | 27.394 | 3.2530 | 4.3 |
| 8 | 15.206 | 5.8219 | 4.9 | 28 | 28.322 | 3.1485 | 10.5 |
| 9 | 15.854 | 5.5855 | 6.3 | 29 | 29.267 | 3.0490 | 10.1 |
| 10 | 16.205 | 5.4652 | 16.0 | 30 | 29.856 | 2.9901 | 4.2 |
| 11 | 16.663 | 5.3158 | 22.8 | 31 | 30.568 | 2.9221 | 6.3 |
| 12 | 17.174 | 5.1589 | 100.0 | 32 | 31.336 | 2.9523 | 5.2 |
| 13 | 17.644 | 5.0224 | 3.8 | 33 | 32.163 | 2.7807 | 23.2 |
| 14 | 18.891 | 4.6936 | 64.3 | 34 | 32.622 | 2.7427 | 7.1 |
| 15 | 19.182 | 4.6232 | 48.5 | 35 | 33.269 | 2.6908 | 8.6 |
| 16 | 19.819 | 4.4759 | 36.4 | 36 | 33.785 | 2.6509 | 5.4 |
| 17 | 20.743 | 4.2787 | 6.4 | 37 | 34.453 | 2.6010 | 17.9 |
| 18 | 21.298 | 4.1685 | 37.9 | 38 | 34.750 | 2.5795 | 13.5 |
| 19 | 22.086 | 4.0214 | 46.7 | 39 | 36.801 | 2.4403 | 6.5 |
| 20 | 22.913 | 3.8781 | 39.0 | 40 | 39.327 | 2.2891 | 4.2 |

In some embodiments of the present disclosure, the crystal form A can also be characterized by a DSC pattern having an onset temperature of 206.05 °C and a peak temperature of 207.18 °C.

In some embodiments of the present disclosure, the differential scanning calorimetry curve of the crystal form A has an endothermic peak at 206.05 °C ± 3 °C.

In some embodiments of the present disclosure, the differential scanning calorimetry curve pattern of the crystal form A is as shown in Fig. 2.

In some embodiments of the present disclosure, the crystal form A can be characterized by a TGA pattern showing a weight loss of 0.07730 % occurred at 158.11 °C, a further weight loss of 0.9855 % occurred at 203.86 °C, and a large weight loss occurred after 203.86 °C.

In some embodiments of the present disclosure, the thermogravimetric analysis curve of the crystal form A has a weight loss of 0.07730 % occurred at 158.11 °C±3 °C, and a weight loss of 1.0628 % occurred at 203.86 °C±3 °C.

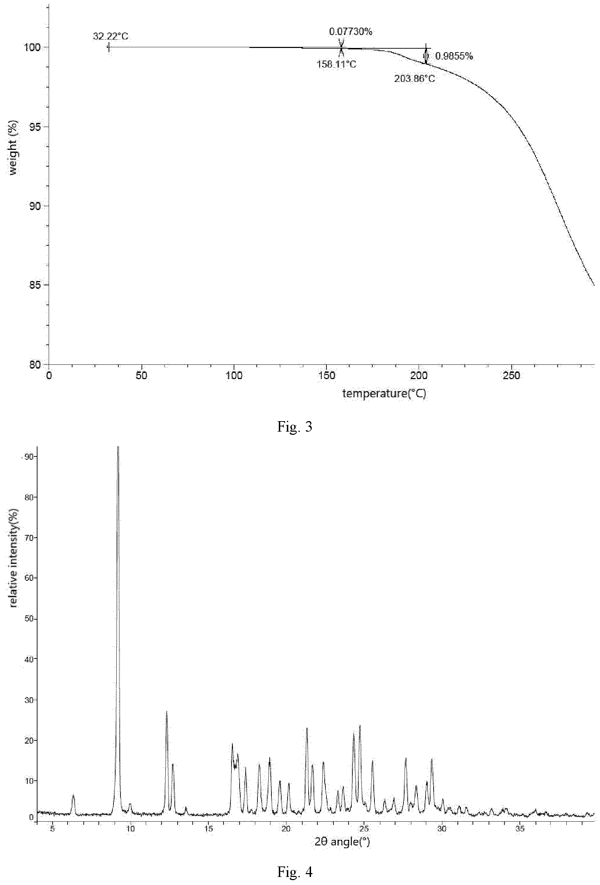

In some embodiments of the present disclosure, the thermogravimetric analysis curve pattern of the crystal form A is as shown in Fig. 3.

The present disclosure also provides a crystal form B of a compound represented by formula (I), the X-ray powder diffraction pattern thereof has characteristic diffraction peaks at the following 2θ angles: 9.19 ± 0.20°, 12.34 ± 0.20° and 16.45 ± 0.20°.

In some embodiments of the present disclosure, the X-ray powder diffraction pattern of the crystal form B has characteristic diffraction peaks at the following 2θ angles: 9.19±0.20°, 12.34±0.20°, 16.45±0.20°, 16.88±0.20°, 18.95±0.20°, 21.34±0.20°, 22.39±0.20° and 24.34±0.20°.

In some embodiments of the present disclosure, the X-ray powder diffraction pattern of the crystal form B has characteristic diffraction peaks at the following 2θ angles: 6.35°, 9.19°, 10.00°, 12.34°, 12.74°, 13.57°, 16.55°, 16.88°, 17.40°, 17.80°, 18.28°, 18.95°, 19.60°, 20.19°, 21.34°, 21.69°, 22.39°, 23.33°, 23.68°, 24.34°, 24.73°, 25.56°, 26.35°, 26.94°, 27.69°, 28.36°, 29.03°, 29.35°, 30.06°, 30.55°, 31.12°, 33.19°, 33.86°, 34.10°, 36.01° and 36.66°.

In some embodiments of the present disclosure, the X-ray powder diffraction pattern of the crystal form B is as shown in Fig. 4.

In some embodiments of the present disclosure, the analytical data of the XRPD pattern of the crystal form B is as shown in Table 2.

**Table 2: Analytical data of the XRPD pattern of the crystal form B of the compound represented by formula (I)**

| **No.** | **2θ Angle (°)** | **d-Spacing (Å)** | **Relative intensity (%)** | **No.** | **2θ Angle (°)** | **d-Spacing (Å)** | **Relative intensity (%)** |
|---|---|---|---|---|---|---|---|
| 1 | 6.348 | 13.9112 | 3.1 | 19 | 23.681 | 3.7540 | 8.0 |
| 2 | 9.190 | 9.6153 | 100.0 | 20 | 24.337 | 3.6543 | 24.1 |
| 3 | 9.995 | 8.8420 | 4.5 | 21 | 24.731 | 3.5969 | 29.0 |
| 4 | 12.344 | 7.1647 | 27.1 | 22 | 25.558 | 3.4824 | 12.0 |
| 5 | 12.738 | 6.9437 | 13.7 | 23 | 26.346 | 3.3801 | 3.8 |
| 6 | 13.570 | 6.5198 | 1.8 | 24 | 26.938 | 3.3071 | 4.7 |
| 7 | 16.545 | 5.3534 | 74.7 | 25 | 27.687 | 3.2193 | 14.5 |
| 8 | 16.881 | 5.2478 | 57.7 | 26 | 28.357 | 3.1448 | 12.4 |
| 9 | 17.395 | 5.0939 | 11.7 | 27 | 29.028 | 3.0735 | 12.3 |
| 10 | 17.800 | 4.9790 | 1.2 | 28 | 29.346 | 3.0409 | 23.3 |
| 11 | 18.280 | 4.8491 | 14.1 | 29 | 30.055 | 2.9708 | 8.6 |
| 12 | 18.951 | 4.6790 | 15.9 | 30 | 30.545 | 2.9243 | 4.1 |
| 13 | 19.603 | 4.5248 | 7.8 | 31 | 31.119 | 2.8716 | 2.1 |
| 14 | 20.190 | 4.3946 | 5.6 | 32 | 33.192 | 2.6969 | 5.6 |
| 15 | 21.335 | 4.1612 | 20.1 | 33 | 33.861 | 2.6451 | 7.6 |
| 16 | 21.691 | 4.0936 | 14.0 | 34 | 34.098 | 2.6272 | 6.8 |
| 17 | 22.385 | 3.9684 | 17.0 | 35 | 36.013 | 2.4918 | 4.0 |
| 18 | 23.328 | 3.8100 | 5.3 | 36 | 36.663 | 2.4491 | 4.2 |

In some embodiments of the present disclosure, the differential scanning calorimetry curve of the crystal form B has endothermic peaks at 136.23 °C±3 °C and 206.26 °C±3 °C, respectively.

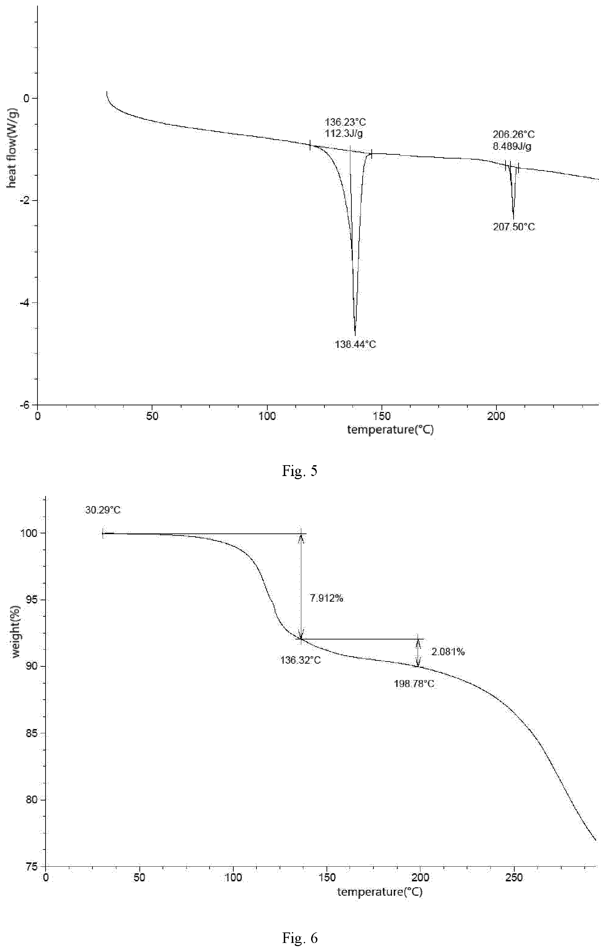

In some embodiments of the present disclosure, the differential scanning calorimetry curve pattern of the crystal form B is as shown in Fig. 5.

In some embodiments of the present disclosure, the crystal form B can be characterized by a TGA pattern showing a weight loss of 7.912 % occurred at 136.32 °C, a further weight loss of 2.081 % occurred at 198.78 °C, and a large weight loss occurred after 198.78 °C.

In some embodiments of the present disclosure, the thermogravimetric analysis curve of the crystal form B has a weight loss of 7.912 % occurred at 136.32 °C ± 3 °C, and a weight loss of 9.993 % occurred at 198.78 °C ± 3 °C.

In some embodiments of the present disclosure, the thermogravimetric analysis curve pattern of the crystal form B is as shown in Fig. 6.

The present disclosure also provides a compound represented by formula (II).

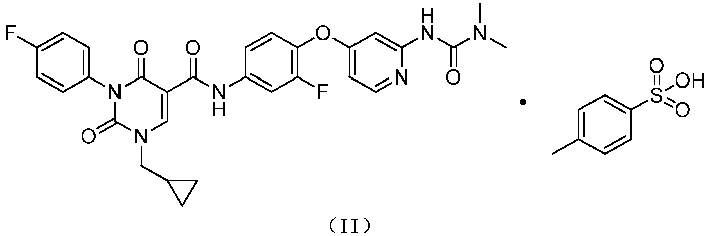

The present disclosure also provides a crystal form C of the compound represented by formula (II), the X-ray powder diffraction pattern thereof has characteristic diffraction peaks at the following 2θ angles: 4.22 ± 0.20°, 14.91 ± 0.20° and 20.75 ± 0.20°.

In some embodiments of the present disclosure, the X-ray powder diffraction pattern of the crystal form C of has characteristic diffraction peaks at the following 2θ angles: 4.22±0.20°, 10.23±0.20°, 14.34±0.20°, 14.91±020°, 19.27±020°, 19.94±020°, 20.75±0.20°, 23.51±0.20°, 28.38±0.20°, 29.03±0.20° and 29.50±0.20°.

In some embodiments of the present disclosure, the X-ray powder diffraction pattern of the crystal form C has characteristic diffraction peaks at the following 2θ angles: 4.22°, 7.18°, 8.26°, 10.23°, 13.47°, 14.34°, 14.91°, 15.68°, 16.06°, 16.48°, 17.07°, 17.67°, 18.12°, 18.65°, 19.27°, 19.94°, 20.35°, 20.75°, 21.55°, 22.23°, 22.48°, 23.51°, 24.73°, 25.34°, 26.07°, 26.35°, 26.94°, 27.25°, 27.63°, 28.38°, 29.03°, 29.5°, 29.96°, 30.57°, 31.24°, 32.03°, 32.91°, 33.59°, 34.32°, 34.94°, 35.79°, 37.69° and 38.28°.

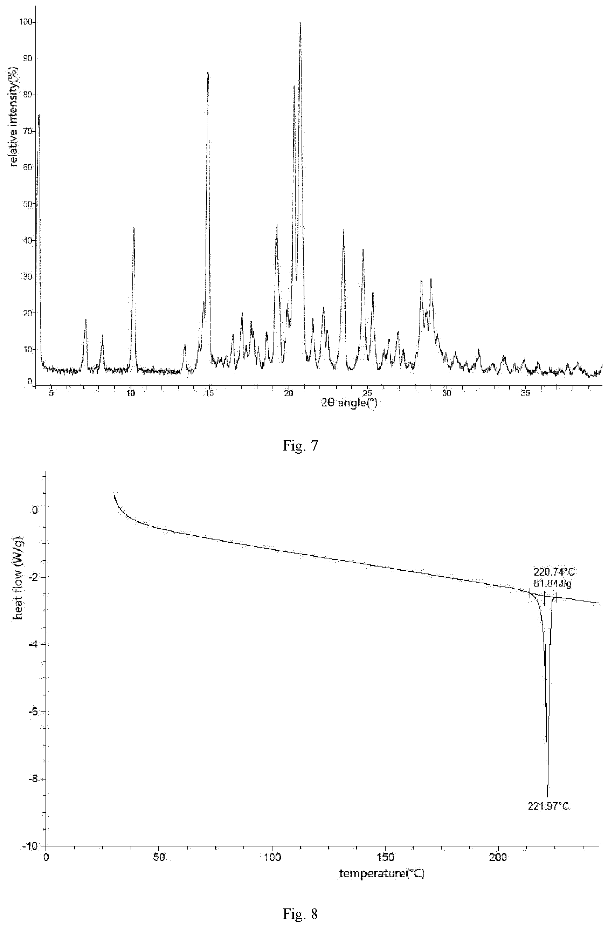

In some embodiments of the present disclosure, the X-ray powder diffraction pattern of the crystal form C is as shown in Fig. 7.

In some embodiments of the present disclosure, the analytical data of the XRPD pattern of the crystal form C is as shown in Table 5.

**Table 3: Analytical data of the XRPD pattern of the crystal form C of the compound represented by formula (II)**

| **No.** | **2θ Angle (°)** | **d-Spacing (Å)** | **Relative intensity (%)** | **No.** | **2θ Angle (°)** | **d-Spacing (Å)** | **Relative intensity (%)** |
|---|---|---|---|---|---|---|---|
| 1 | 4.216 | 20.9419 | 51.4 | 23 | 24.731 | 3.5969 | 29.9 |
| 2 | 7.176 | 12.3090 | 9.5 | 24 | 25.342 | 3.5116 | 16.3 |
| 3 | 8.261 | 10.6942 | 8.1 | 25 | 26.072 | 3.4149 | 4.2 |
| 4 | 10.231 | 8.6393 | 28.0 | 26 | 26.351 | 3.3795 | 7.2 |
| 5 | 13.465 | 6.5707 | 402 | 27 | 26.937 | 3.3071 | 9.3 |
| 6 | 14.337 | 6.1727 | 60. | 28 | 27.253 | 3.2696 | 5.6 |
| 7 | 14.909 | 5.9373 | 63.3 | 29 | 27.634 | 3.2253 | 2.3 |
| 8 | 15.683 | 5.6460 | 4.3 | 30 | 28.382 | 3.1420 | 51.3 |
| 9 | 16.061 | 5.5139 | 3.4 | 31 | 29.030 | 3.0733 | 69.2 |
| 10 | 16.483 | 5.3734 | 2.9 | 32 | 29.502 | 3.0252 | 47.6 |
| 11 | 17.073 | 5.1893 | 10.8 | 33 | 29.957 | 2.9803 | 7.7 |
| 12 | 17.666 | 5.0163 | 16.4 | 34 | 30.566 | 2.9223 | 3.3 |
| 13 | 18.121 | 4.8913 | 2.8 | 35 | 31.241 | 2.8607 | 3.0 |
| 14 | 18.654 | 4.7527 | 4.7 | 36 | 32.031 | 2.7919 | 4.6 |
| 15 | 19.266 | 4.6031 | 31.6 | 37 | 32.914 | 2.7190 | 2.8 |
| 16 | 19.938 | 4.4496 | 25.0 | 38 | 33.590 | 2.6658 | 3.9 |
| 17 | 20.353 | 4.3596 | 71.9 | 39 | 34.316 | 2.6111 | 2.1 |
| 18 | 20.746 | 4.2780 | 100.0 | 40 | 34.941 | 2.5658 | 3.7 |
| 19 | 21.554 | 4.1194 | 10.6 | 41 | 35.788 | 2.5069 | 1.5 |
| 20 | 22.226 | 3.9964 | 16.4 | 42 | 37.687 | 2.3849 | 3.7 |
| 21 | 22.480 | 3.9517 | 13.7 | 43 | 38.282 | 2.3492 | 5.1 |
| 22 | 23.506 | 3.7816 | 27.9 | | | | |

In some embodiments of the present disclosure, the crystal form C can also be characterized by a DSC pattern having an onset temperature of 220.74 °C and a peak temperature of 221.97 °C.

In some embodiments of the present disclosure, the differential scanning calorimetry curve of the crystal form C has an endothermic peak at 220.74 °C ± 3 °C.

In some embodiments of the present disclosure, the differential scanning calorimetry curve pattern of the crystal form C is as shown in Fig. 8.

In some embodiments of the present disclosure, the crystal form C can be characterized by a TGA pattern showing a weight loss of 0.004784 % occurred at 159.80 °C, and a large weight loss occurred after 159.80 °C.

In some embodiments of the present disclosure, the thermogravimetric analysis curve of the crystal form C has a weight loss of 0.004784 % occurred at 159.80 °C±3 °C.

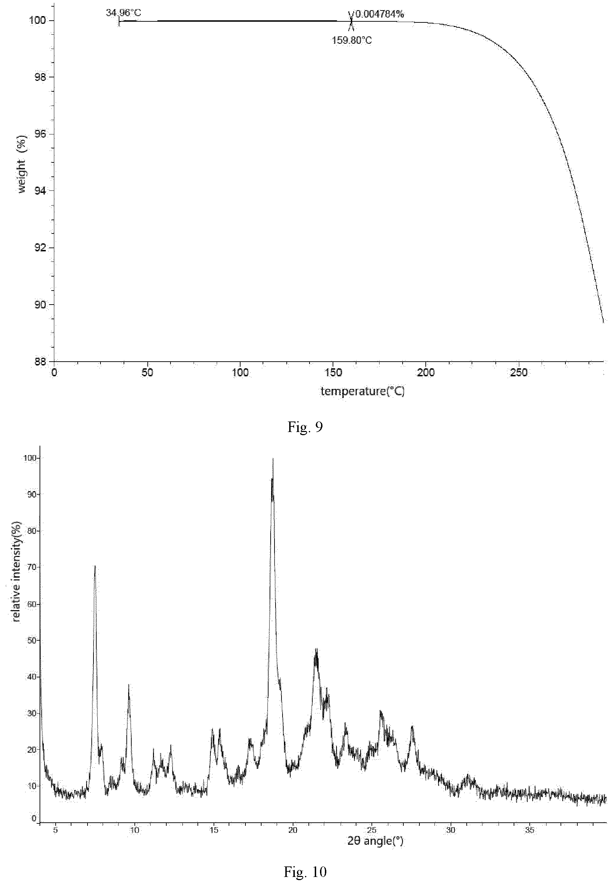

In some embodiments of the present disclosure, the thermogravimetric analysis curve pattern of the crystal form C is as shown in Fig. 9.

In some embodiments of the present disclosure, the infrared spectrogram pattern of the crystal form C includes characteristic absorption peaks at 3248 cm⁻¹±5 cm⁻¹, 3207 cm⁻¹± 5cm⁻¹, 3096 cm⁻¹±5 cm⁻¹, 3064 cm⁻¹±5 cm⁻¹, 3000 cm⁻¹±5 cm⁻¹, 1690±2 cm⁻¹, 1650±2 cm⁻¹, 1609±2 cm⁻¹, 1582±2 cm⁻¹, 1509±2 cm⁻¹, 1208±2 cm⁻¹, 1176±2 cm⁻¹, 1031±2 cm⁻¹ and 1009±2 cm⁻¹.

The present disclosure also provides a compound represented by formula (III).

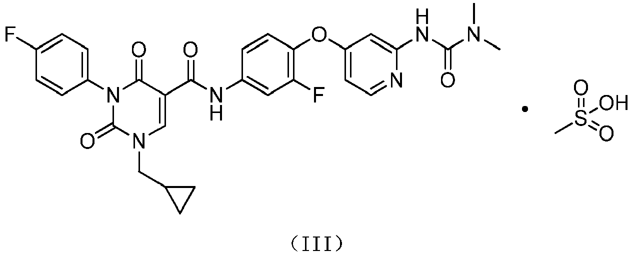

The present disclosure also provides a crystal form D of the compound represented by formula (III), the X-ray powder diffraction pattern thereof has characteristic diffraction peaks at the following 2θ angles: 7.49 ± 0.20°, 9.64 ± 0.20°, and 19.23 ± 0.20°.

In some embodiments of the present disclosure, the X-ray powder diffraction pattern of the crystal form D has characteristic diffraction peaks at the following 2θ angles: 7.49±0.20°, 9.64±0.20°, 18.75±0.20°, 19.23±0.20°, 20.93±0.20°, 21.55±0.20° and 22.17±0.20°.

In some embodiments of the present disclosure, the X-ray powder diffraction pattern of the crystal form D has characteristic diffraction peaks at the following 2θ angles: 7.49°, 7.89°, 8.50°, 9.17°, 9.64°, 11.20°, 11.67°, 12.28°, 14.93°, 15.40°, 17.35°, 18.75°, 19.23°, 20.93°, 21.55°, 22.17°, 23.31°, 24.12°, 24.88°, 25.58°, 26.53°, 27.53° and 31.10°.

In some embodiments of the present disclosure, the X-ray powder diffraction pattern of the crystal form D is as shown in Fig. 10.

In some embodiments of the present disclosure, the analytical data of the XRPD pattern of the crystal form D is as shown in Table 4.

**Table 4: Analytical data of the XRPD pattern of the crystal form D of the compound represented by formula (III)**

| No. | 2θ Angle (°) | d-Spacing (Å) | Relative intensity (%) | No. | 2θ Angle (°) | d-Spacing (Å) | Relative intensity (%) |
|---|---|---|---|---|---|---|---|
| 1 | 7.491 | 11.7911 | 29.8 | 13 | 19.226 | 4.6126 | 100.0 |
| 2 | 7.887 | 11.2001 | 8.0 | 14 | 20.925 | 4.2417 | 44.5 |
| 3 | 8.497 | 10.3971 | 4.2 | 15 | 21.554 | 4.1195 | 51.8 |
| 4 | 9.165 | 9.6407 | 11.1 | 16 | 22.166 | 4.0070 | 37.5 |
| 5 | 9.642 | 9.1650 | 21.5 | 17 | 23.309 | 3.8130 | 14.1 |
| 6 | 11.198 | 7.8951 | 7.9 | 18 | 24.119 | 3.6868 | 9.1 |
| 7 | 11.671 | 7.5762 | 10.8 | 19 | 24.876 | 3.5763 | 5.8 |
| 8 | 12.284 | 7.1993 | 12.4 | 20 | 25.578 | 3.4798 | 26.1 |
| 9 | 14.926 | 5.9305 | 11.7 | 21 | 26.525 | 3.3576 | 20.2 |
| 10 | 15.398 | 5.7497 | 13.5 | 22 | 27.531 | 3.2372 | 3.5 |
| 11 | 17.353 | 5.1062 | 13.5 | 23 | 31.098 | 2.8735 | 4.1 |
| 12 | 18.753 | 4.7280 | 73.0 | | | | |

In some embodiments of the present disclosure, the crystal form D can also be characterized by a DSC pattern having an onset temperature of 223.59 °C and a peak temperature of 226.43 °C.

In some embodiments of the present disclosure, the differential scanning calorimetry curve of the crystal form D has an endothermic peak at 223.59 °C ± 3 °C.

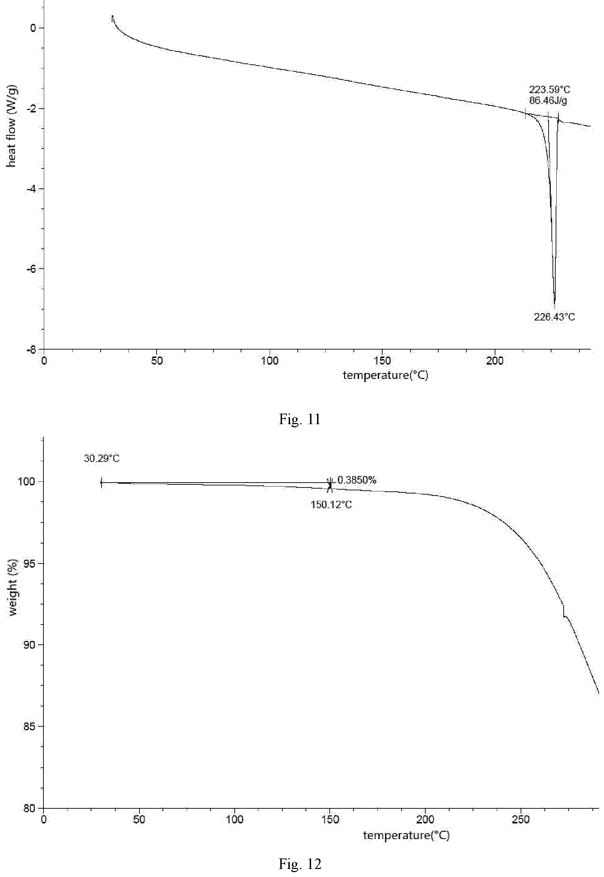

In some embodiments of the present disclosure, the differential scanning calorimetry curve pattern of the crystal form D is as shown in Fig. 11.

In some embodiments of the present disclosure, the crystal form D can be characterized by a TGA pattern showing a weight loss of 0.3850 % occurred at 150.12 °C, and a large weight loss occurred after 150.12 °C.

In some embodiments of the present disclosure, the thermogravimetric analysis curve pattern of the crystal form D is as shown in Fig. 12.

The present disclosure also provides a compound represented by formula (IV).

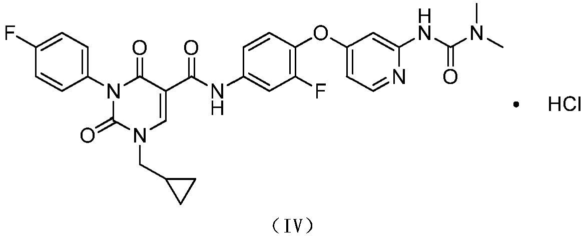

The present disclosure also provides a crystal form E of the compound represented by formula (IV), the X-ray powder diffraction pattern thereof has characteristic diffraction peaks at the following 2θ angles: 6.94 ± 0.20°, 10.00 ± 0.20° and 11.73 ± 0.20°.

In some embodiments of the present disclosure, the X-ray powder diffraction pattern of the crystal form E has characteristic diffraction peaks at the following 2θ angles: 5.85±0.20°, 6.94±0.20°, 10.00±0.20°, 11.73±0.20°, 15.82±0.20°, 17.10±0.20°, 20.39±0.20° and 23.74±0.20°.

In some embodiments of the present disclosure, the crystal form E 5.85°, 6.94°, 10.00°, 11.73°, 13.83°, 14.41°, 15.82°, 16.38°, 17.10°, 17.47°, 18.06°, 18.95°, 20.00°, 20.39°, 20.88°, 22.25°, 23.74°, 24.91°, 25.48°, 26.39°, 27.57°, 29.86°, 30.49°, 32.62°, 35.79° and 37.14°.

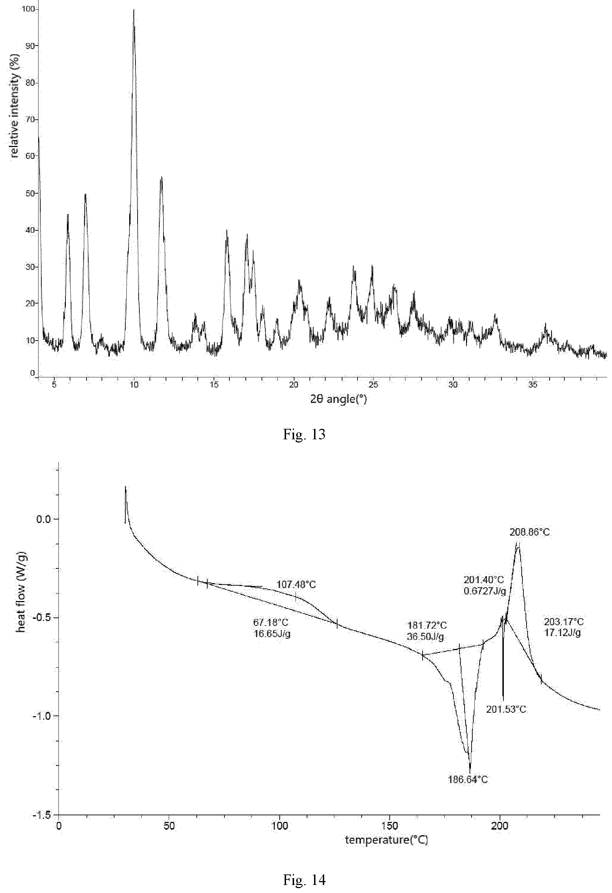

In some embodiments of the present disclosure, the X-ray powder diffraction pattern of the crystal form E is as shown in Fig. 13.

In some embodiments of the present disclosure, the analytical data of the XRPD pattern of the crystal form E is as shown in Table 5.

**Table 5: Analytical data of the XRPD pattern of the crystal form E of the compound represented by formula (IV)**

| **No.** | **2θ Angle (°)** | **d-Spacing (Å)** | **Relative intensity (%)** | **No.** | **2θ Angle (°)** | **d-Spacing (Å)** | **Relative intensity (%)** |
|---|---|---|---|---|---|---|---|
| 1 | 5.854 | 15.0841 | 26.1 | 14 | 20.392 | 4.3515 | 31.8 |
| 2 | 6.940 | 12.7257 | 39.0 | 15 | 20.881 | 4.2506 | 14.8 |
| 3 | 9.996 | 8.8414 | 100.0 | 16 | 22.248 | 3.9925 | 7.5 |
| 4 | 11.733 | 7.5364 | 60.6 | 17 | 23.744 | 3.7442 | 33.3 |
| 5 | 13.825 | 6.4002 | 12.8 | 18 | 24.909 | 3.5717 | 47.6 |
| 6 | 14.412 | 6.1409 | 7.9 | 19 | 25.481 | 3.4928 | 20.6 |
| 7 | 15.816 | 5.5988 | 36.2 | 20 | 26.386 | 3.3750 | 25.3 |
| 8 | 16.383 | 5.4062 | 11.8 | 21 | 27.571 | 3.2326 | 11.0 |
| 9 | 17.096 | 5.1822 | 47.3 | 22 | 29.861 | 2.9896 | 7.4 |
| 10 | 17.472 | 5.0715 | 37.5 | 23 | 30.485 | 2.9298 | 8.7 |
| 11 | 18.063 | 4.9069 | 8.4 | 24 | 32.618 | 2.7430 | 11.0 |
| 12 | 18.952 | 4.6788 | 4.0 | 25 | 35.794 | 2.5066 | 15.7 |
| 13 | 19.998 | 4.4363 | 21.8 | 26 | 37.140 | 2.4188 | 7.7 |

In some embodiments of the present disclosure, the differential scanning calorimetry curve of the crystal form E has exothermic peaks with onset at 67.18 °C±3 °C and 203.17 °C±3 °C, and has endothermic peaks with onset at 181.72 °C±3 °C and 201.40 °C±3 °C.

In some embodiments of the present disclosure, the differential scanning calorimetry curve pattern of the crystal form E is as shown in Fig. 14.

In some embodiments of the present disclosure, the thermogravimetric analysis curve of the crystal form E has a weight loss of 0.5018 % occurred at 52.80 °C±3 °C, and a weight loss of 4.4958 % occurred at 173.60 °C±3 °C, and a weight loss of 5.8808 % occurred at 210.40 °C±3 °C.

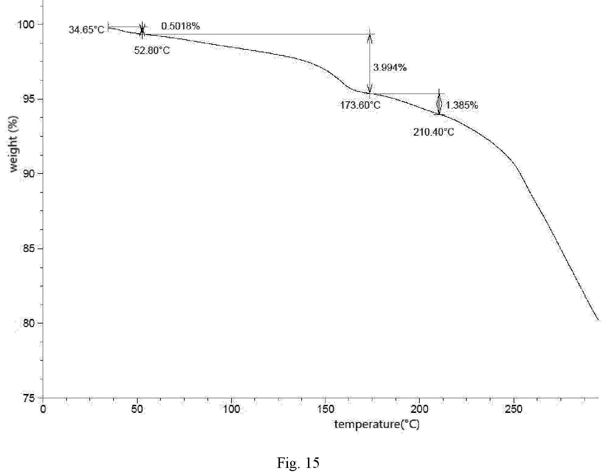

In some embodiments of the present disclosure, the thermogravimetric analysis curve pattern of the crystal form E is as shown in Fig. 15.

It should be noted that, in the X-ray powder diffraction pattern, the position of the peak or the relative intensity of the peak may be different due to factors such as measuring instrument, measuring method/condition, etc. For any specific crystal form, there may be errors in the position of peaks, and the measurement error of 2θ value may be ±0.50°, ±0.30° or ±0.20°. Therefore, the error should be taken into account when determining each crystal type, and is within the scope of the present disclosure within the error.

It should be noted that, for the same crystal form, the position of the endothermic peak of DSC may be different due to factors such as measuring instrument, measuring method/condition, etc. For any specific crystal form, there may be an error in the position of endothermic peak, which may be ± 5 °C, ± 3 °C or ± 2 °C. Therefore, the error should be taken into account when determining each crystal type, and is within the scope of the present disclosure within the error.

It should be noted that, for the same crystal form, the position of TGA weight loss temperature may be different due to factors such as measuring instrument, measuring method/condition, etc. For any specific crystal form, there may be an error in the position of weight loss temperature, which may be ± 5°C, ± 3°C or ± 2°C. Therefore, the error should be taken into account when determining each crystal type, and is within the scope of the present disclosure within the error.

### Technical effect

The crystal forms and salt forms of the compounds of the present disclosure have strong inhibitory activity on c-MET and AXL enzymes, and show better inhibitory activity on MKN45 cells, good tumor inhibitory effect, and good stability, not easy to absorb moisture, easy to prepare.

### Definition and description

Unless otherwise indicated, the following terms and phrases used in this document are intended to have the following meanings. A specific term or phrase should not be considered indefinite or unclear in the absence of a particular definition, but should be understood in the ordinary sense. When a trade name appears herein, it is intended to refer to its corresponding commodity or active ingredient thereof.

The intermediate compounds of the present disclosure can be prepared by various synthetic methods known to those skilled in the art, including the embodiments described below, the embodiments formed by combining the embodiments described below with other chemical synthesis methods, and equivalent alternatives well-known to those skilled in the art. Preferred embodiments include, but are not limited to, the embodiments of the present disclosure.

The chemical reactions of the embodiments of the present disclosure are carried out in a suitable solvent, and the solvent should be suitable for the chemical change, and the reagents and materials required therefor of the present disclosure. In order to obtain the compounds of the present disclosure, it is sometimes necessary for those skilled in the art to modify or select the synthetic steps or reaction schemes based on the existing embodiments.

The present disclosure will be specifically described below by way of embodiments, but the scope of the present disclosure is not limited thereto.

All solvents used in the present disclosure are commercially available and can be directly used without further purification.

**The present disclosure employs the following abbreviations:**
DIPEA: *N,N*-diisopropylethylamine
THF: tetrahydrofuran
TBTU: *O-*benzotriazole-*N,N,N',N*'-tetramethyluronium tetrafluoroborate
Compounds are named according to conventional naming principles in the art or by ChemDraw^{®} software, and the commercially available compounds use their vendor directory names.
**X-ray powder diffraction (XRPD) method in the present disclosure**
Instrument model: Bruker D8 Advance X-ray diffractometer
Detection method: about 10-20 mg of the sample was used for XRPD detection.
The detailed XRPD parameters were as follows:
X-ray tube: Cu, kα, (λ=1.54056Å)
X-ray tube voltage: 40 kV, X-ray tube current: 40 mA
Divergence slit: 0.60 mm
Detector slit: 10.50 mm
Anti-scattering slit: 7.10 mm
Scanning range: 3 or 4-40 deg
Step size: 0.02 deg
Step time: 0.12 second
Rotation speed of sample tray: 15 rpm
**Differential Scanning Calorimeter (DSC) method in the present disclosure**
Instrument model: TADSCQ2000 differential scanning calorimeter
Detection method: 0.5-1 mg of the sample was placed in a DSC aluminum crucible for testing, under the condition of 50 mL/min N₂ at a heating rate of 10 °C/min, the sample was heated from room temperature (25 °C) to 300 °C, or 350 °C.
**Thermal Gravimetric Analyzer (TGA) method in the present disclosure**
Instrument model: TAQ5000 thermal gravimetric analyzer
Detection method: 2-5 mg of the sample was placed in a TGA platinum crucible for testing, under the condition of 25 mL/min N₂ at a heating rate of 10/min, the sample was heated from room temperature (25 °C) to 300 °C, 350 °C or until a weight loss of 20 %.
**Dynamic Vapor Sorption (DVS) instrument in the present disclosure**
Instrument model: DVS Advantage-1 (SMS)
Detection condition: about 10-15 mg of the sample was used for DVS detection.
Equilibrium: dm/dt=0.01%/min: (time: 10 min, longest: 180 min)
Drying: 0%RH, 120 min
RH(%) gradient for testing: 10%
RH(%) gradient range for testing: 0%-90%-0%
The hygroscopicity was evaluated using the judgment criterias in the following Table 6:

**Tabel 6: Judgment criterias for hygroscopicity**

| Classification of hygroscopicity | Hygroscopic weight gain^{∗} |
|---|---|
| Deliquescence | Absorbing sufficient water to form liquid |
| Highly hygroscopic | Hygroscopic weight gain ≥ 15% |
| Hygroscopic | 2%≤Hygroscopic weight gain <15% |
| Slightly hygroscopic | 0.2%≤Hygroscopic weight gain <2% |
| Non- or almost non- hygroscopic | Hygroscopic weight gain < 0.2% |

| | |
|---|---|
| ^{∗}Hygroscopic weight gain at 25 °C/80% RH. | |

### High Performance Liquid Chromatography (HPLC) method in the present disclosure

### Instrument model: Agilent 1200 High Performance Liquid Chromatography

The analysis method was as follows:

**Table 7: HPLC analysis method for related substance content test**

| Instrument | Agilent 1200 High Performance Liquid Chromatography | | |
|---|---|---|---|
| Chromatographic column | Eclipse Plus C18 3.5um 4.6^{∗}150 mm | | |
| Mobile phase A | 0.0375% Trifluoroacetic acid aqueous solution | | |
| Mobile phase B | 0.0188% Trifluoroacetic acid acetonitrile solution | | |
| Flow rate | 1.0 mL/min | | |
| Injection volume | 1.5 µL | | |
| Detection wavelength | 220 nm/254 nm | | |
| Column temperature | 40 °C | | |
| Diluent | Acetonitrile | | |

| | Duration (min) | Mobile phase A (%) | Mobile phase B (%) |
|---|---|---|---|
| Gradient elution program | 0.00 | 100 | 0 |
| | 40.00 | 40 | 60 |
| | 55.00 | 0 | 100 |
| | 60.00 | 0 | 100 |

### Brief description of the drawings

Fig. 1 is the XRPD pattern of the crystal form A of the compound represented by formula (I).
Fig. 2 is the DSC pattern of the crystal form A of the compound represented by formula (I).
Fig. 3 is the TGA pattern of the crystal form A of the compound represented by formula (I).
Fig. 4 is the XRPD pattern of the crystal form B of the compound represented by formula (I).
Fig. 5 is the DSC pattern of the crystal form B of the compound represented by formula (I).
Fig. 6 is the TGA pattern of the crystal form B of the compound represented by formula (I).
Fig. 7 is the XRPD pattern of the crystal form C of the compound represented by formula (II).
Fig. 8 is the DSC pattern of the crystal form C of the compound represented by formula (II).
Fig. 9 is the TGA pattern of the crystal form C of the compound represented by formula (II).
Fig. 10 is the XRPD pattern of the crystal form D of the compound represented by formula (III).
Fig. 11 is the DSC pattern of the crystal form D of the compound represented by formula (III).
Fig. 12 is the TGA pattern of the crystal form D of the compound represented by formula (III).
Fig. 13 is the XRPD pattern of the crystal form E of the compound represented by formula (IV).
Fig. 14 is the DSC pattern of the crystal form E of the compound represented by formula (IV).
Fig. 15 is the TGA pattern of the crystal form E of the compound represented by formula (IV).

### Detailed description of the embodiment

For better understanding of the content of the present disclosure, the present disclosure is described in detail through the embodiments, but the embodiments do not mean any limitation on the present disclosure.

For better understanding of the content of the present disclosure, the present disclosure is described in detail through the embodiments, but the embodiments do not mean any limitation on the present disclosure.

### Embodiment 1: Preparation of the crystal form A of the compound represented by Formula (I)

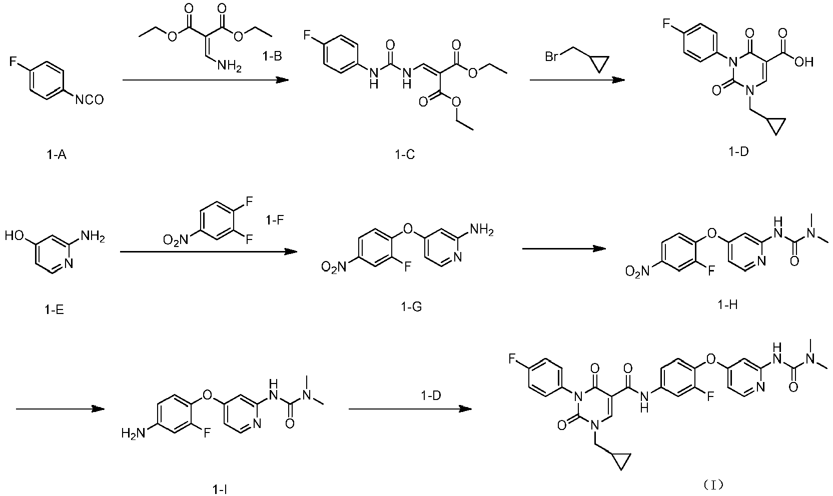

### Preparation of 1-C:

Under nitrogen protection, DIPEA (202.56 g, 1.57 mol) and compound 1-B (251.66 g, 1.34 mol) were added to a solution of compound 1-A (201.93 g, 1.47 mol) in toluene (2 L) with stirring. The reaction mixture was reacted at 100 °C for 16 hours. The reaction mixture was naturally cooled to room temperature and stirred for 16 hours, filtered, and the filter cake was collected to obtain intermediate 1-C. LCMS (ESI) m/z: 347.0 [M+Na]⁺, ¹HNMR (400 MHz, DMSO-d6) δ ppm 1.26 (dt, J=12.26, 7.08 Hz, 6 H) 4.15 (q, J=7.09 Hz, 2 H) 4.24 (q, J=7.13 Hz, 2 H) 7.12 - 7.26 (m, 2 H) 7.44 - 7.59 (m, 2 H) 8.47 (d, J=12.23 Hz, 1 H) 10.40 (s, 1 H) 10.57 (br d, J=12.47 Hz, 1 H).

### Preparation of 1-D:

Under nitrogen protection, potassium carbonate (169.94 g, 1.23 mol) was added to a solution of intermediate 1-C (197.73 g, 0.61 mol) in ethanol (1 L). The reaction mixture was reacted at 75°C for 2 hours, and bromomethylcyclopropane (166.63 g, 1.23 mol) was added thereto. The reaction mixture was reacted at 75°C for 16 hours, and water (1 L) was added thereto. The reaction mixture was reacted at 75°C for 16 hours. The reaction mixture was concentrated under reduced pressure to remove ethanol, the residue was extracted with ethyl acetate (500 mL^{∗}2), the aqueous phase was collected. 12M hydrochloric acid was added to adjust the pH to 1, then the mixture was filtered, and the filter cake was collected and dried to obtain intermediate 1-D. LCMS (ESI) m/z: 304.9 [M+H]⁺; ¹H NMR (400 MHz, DMSO-d6) δ ppm 0.37 - 0.45 (m, 2 H) 0.50 - 0.60 (m, 2 H) 1.07 - 1.35 (m, 1 H) 3.80 (d, J=7.21 Hz, 2 H) 7.23 - 7.51 (m, 4 H) 8.83 (s, 1 H) 12.63 (br s, 1 H).

### Preparation of 1-G:

Under nitrogen protection, a solution of compound 1-E (50.09 g, 0.45 mol), compound 1-F (77.47 g, 0.49 mol) and potassium carbonate (66.06 g, 0.48 mol) in acetonitrile (250 mL) was heated to 50°C and reacted for 16 hours. 750 mL of water was added, the reaction mixture was stirred at room temperature for 48 hours, then filtered, and the filter cake was collected and dried to obtain intermediate 1-G. LCMS (ESI) m/z: 250.0 [M+H]⁺; ¹NMR(400 MHz, DMSO-d6) δ ppm 6.00 (d, J=2.32 Hz, 1 H) 6.10 (s, 2 H) 6.26 (dd, J=5.75, 2.32 Hz, 1 H) 7.45 - 7.59 (m, 1 H) 7.90 (d, J=5.75 Hz, 1 H) 8.11 - 8.24 (m, 1 H) 8.39 (dd, J=10.45, 2.75 Hz, 1 H).

### Preparation of 1-H:

Under nitrogen protection, phenyl chloroformate (51 mL, 0.40 mol) was added dropwise to a mixture of compound 1-G (50.35 g, 0.20 mol) and DIPEA (105 mL, 0.61 mol). After the reaction mixture was reacted at 0°C for 3 hours, 300 mL of dimethylamine tetrahydrofuran solution (2 moles per liter) was added, and the reaction mixture was reacted at 50°C for 16 hours. The reaction mixture was cooled to room temperature and stirred for 16 hours, filtered, and the filtrate was concentrated under reduced pressure, and the residue was purified by column chromatography to obtain intermediate 1-H. LCMS (ESI) m/z: 321.0 [M+H]⁺; ¹H NMR (400 MHz, DMSO-d6) δ ppm 2.91 (s, 6 H) 6.75 (dd, J=5.69, 2.38 Hz, 1 H) 7.53 (d, J=2.32 Hz, 1 H) 7.56 - 7.63 (m, 1 H) 8.16 - 8.24 (m, 2 H) 8.43 (dd, J=10.52, 2.69 Hz, 1 H) 9.07 (s, 1 H)).

### Preparation of 1-I:

Under nitrogen protection, iron powder (13.08 g, 234.2 mmol) was added to a mixture of compound 1-H (15 g, 46.8 mmol), acetic acid (14.06 g, 234.12 mmol), THF (150 mL) and water (30 mL), and the reaction mixture was reacted for 16 hours at room temperature. The reaction mixture was filtered, the filtrate was concentrated under reduced pressure. The residue was dissolved by adding 500 mL of ethyl acetate and then washed with saturated saline (300 mL^{∗}2). The organic phase was concentrated under reduced pressure, and the residue was purified by column chromatography to obtain intermediate 1-I. LCMS (ESI) m/z: 291.1 [M+H]⁺; ¹H NMR (400 MHz, DMSO-d6) δ ppm 2.91 (s, 6 H) 6.75 (dd, J=5.69, 2.38 Hz, 1 H) 7.53 (d, J=2.32 Hz, 1 H) 7.56 - 7.63 (m, 1 H) 8.16 - 8.24 (m, 2 H) 8.43 (dd, J=10.52, 2.69 Hz, 1 H) 9.07 (s, 1 H)).

### Preparation of the crystal form A of the compound represented by formula (I):

Under nitrogen protection, TBTU (6.33 g, 19.7 mmol) was added to a solution of compound 1-D (5.01 g, 16.4 mmol) and DIPEA (6.37 g, 49.3 mmol) in DMF (50 mL), and after the mixture was stirred for 0.5 hours, compound 1-I (5.03 g, 17.2 mmol) was added thereto, and the reaction mixture was reacted at room temperature for 16 hours. Then 50 mL of water was added dropwise to the reaction reaction, the mixture was stirred for 2 hours at room temperature and filtered, and the filter cake was collected, dried, and recrystallized by ethyl acetate to obtain compound represented by formula (I), which was detected by XRPD (Fig.1) as crystal form A of the compound represented by formula (I). LCMS (ESI) m/z: 577.1 [M+H]⁺; ¹H NMR (400 MHz, DMSO-d6) δ ppm 0.39 - 0.48 (m, 2 H) 0.52 - 0.61 (m, 2 H) 1.18 - 1.33 (m, 1 H) 2.89 (s, 6 H) 3.86 (d, J=7.21 Hz, 2 H) 6.61 (dd, J=5.75, 2.45 Hz, 1 H) 7.29 - 7.41 (m, 4 H) 7.41 - 7.47 (m, 2 H) 7.49 (dd, J=8.86, 1.28 Hz, 1 H) 7.97 (dd, J=12.96,2.45 Hz, 1 H) 8.12 (d, J=5.75 Hz, 1 H) 8.81 - 9.01 (m, 2 H) 11.01 (s, 1 H).

### Embodiment 2: Preparation of the crystal form B of the compound represented by formula (I)

100 mg of crystal form A of the compound represented by formula (I) was weighed and added to a 40 mL vial, then 2 mL of acetone was added thereto. The sample was placed on a magnetic stirrer (40 °C) and stirred for 16 hours, filtered, and the obtained solid was dried at 40 °C under vacuum, which was detected by XRPD (Fig.4) as crystal form B of the compound represented by formula (I).

### Embodiment 3: Preparation of the crystal form C of the compound represented by formula (II)

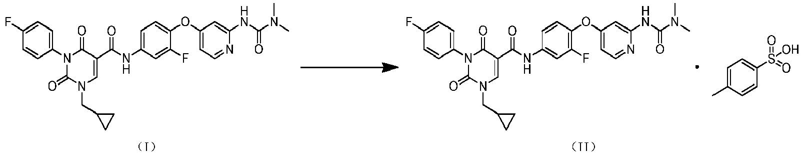

1g of crystal form A of the compound represented by formula (I) was weighted and added to a 40 mL vial, then 20 mL of THF was added thereto. The obtained sample was placed on a magnetic stirrer (40 °C) and stirred for 10 min, and then an appropriate amount of *p*-toluenesulfonic acid (the molar ratio of the compound represented by formula (I) to *p*-toluenesulfonic acid was 1:1.05, added after diluting with THF) was slowly added thereto, and the reaction mixture was dissolved until clear. The sample was placed on a magnetic stirrer (40 °C) and was stirred for 16 hours. A white solid was precipitated from the reaction mixture. Then the mixture was filtered, and the obtained solid was dried at 40 °C overnight in a vacuum drying oven to obtain compound represented by formula (II), which was detected by XRPD (Fig 7) as crystal form C of the compound represented by formula (II). ¹H NMR (400 MHz, DMSO-d6) δ = 11.07 (s, 1H), 10.07 (br s, 1H), 8.92 (s, 1H), 8.28 (d, J = 6.8 Hz, 1H), 8.07 (dd, J = 2.4, 12.9 Hz, 1H), 7.60 (dd, J = 1.5, 8.9 Hz, 1H), 7.52 - 7.41 (m, 5H), 7.41 - 7.33 (m, 2H), 7.18 - 7.08 (m, 3H), 7.04 (d, J = 2.1 Hz, 1H), 3.87 (d, J = 7.2 Hz, 2H), 2.97 (s, 6H), 2.29 (s, 3H), 1.32 -1.18 (m, 1H), 0.63 - 0.52 (m, 2H), 0.49 - 0.39 (m, 2H).

### Embodiment 4: Preparation of the crystal form D of the compound represented by formula (III)

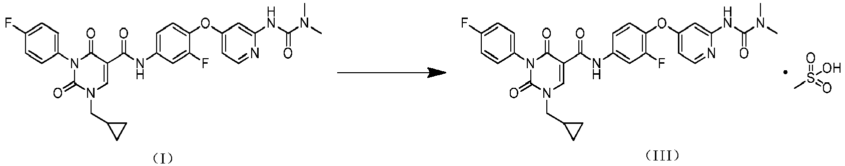

1 g of crystal form A of the compound represented by formula (I) was weighted and added to a 40 mL vial, then 20 mL of THF was added thereto. The obtained sample was placed on a magnetic stirrer (40 °C) and stirred for 10 min, and then an appropriate amount of methanesulfonic acid (the molar ratio of the compound represented by formula (I) to methanesulfonic acid was 1:1.05, added after diluting with THF) was slowly added, and the reaction mixture was dissolved until clear. The sample was placed on a magnetic stirrer (40 °C) and was stirred for 16 hours. A white solid was precipitated from the reaction mixture. Then the mixture was filtered, and the obtained solid was dried at 40 °C overnight in a vacuum drying oven to obtain compound represented by formula (III), which was detected by XRPD (Fig. 10) as crystal form D of the compound represented by formula (III). ¹H NMR (400 MHz, DMSO-d6) δ = 11.07 (s, 1H), 10.09 (br s, 1H), 8.92 (s, 1H), 8.28 (d, J = 7.0 Hz, 1H), 8.07 (dd, J = 2.4, 12.8 Hz, 1H), 7.60 (dd, J = 1.4, 9.0 Hz, 1H), 7.53 - 7.41 (m, 3H), 7.41 - 7.31 (m, 2H), 7.14 (br d, J = 6.4 Hz, 1H), 7.04 (d, J = 2.2 Hz, 1H), 3.87 (d, J = 7.1 Hz, 2H), 2.98 (s, 6H), 2.31 (s, 3H), 1.34 - 1.18 (m, 1H), 0.63 - 0.53 (m, 2H), 0.49 - 0.38 (m, 2H).

### Embodiment 5: Preparation of the crystal form E of the compound represented by formula (IV)

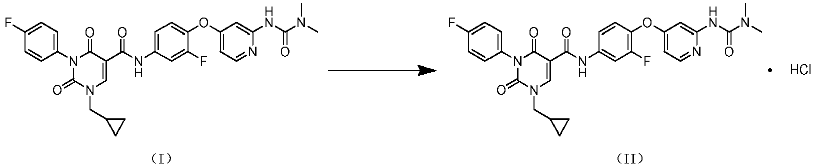

1 g of crystal form A of the compound represented by formula (I) was weighted and added to a 40 mL vial, then 20 mL of THF was added thereto. The obtained sample was placed on a magnetic stirrer (40 °C) and stirred for 10 min, and then an appropriate amount of hydrochloric acid (the molar ratio of the compound represented by formula (I) to hydrochloric acid was 1:1.05, added after diluting with THF) was slowly added, and the reaction mixture was dissolved until clear. The sample was placed on a magnetic stirrer (40 °C) and was stirred for 16 hours. A white solid was precipitated from the reaction mixture. Then the mixture was filtered, and the obtained solid was dried at 40 °C overnight in a vacuum drying oven to obtain compound represented by formula (IV),which was detected by XRPD (Fig. 13) as crystal form E of the compound represented by formula (IV). ¹H NMR (400 MHz, DMSO-d6) δ = 11.07 (s, 1H), 10.25 (br s, 1H), 8.92 (s, 1H), 8.28 (d, J = 6.8 Hz, 1H), 8.06 (dd, J = 2.4, 12.9 Hz, 1H), 7.64 - 7.54 (m, 1H), 7.51 - 7.41 (m, 3H), 7.41 - 7.33 (m, 2H), 7.22 (d, J = 2.4 Hz, 1H), 7.09 (br d, J = 5.3 Hz, 1H), 3.87 (d, J = 7.1 Hz, 2H), 2.98 (s, 6H), 1.34 - 1.16 (m, 1H), 0.67 - 0.51 (m, 2H), 0.48 - 0.37 (m, 2H).

### Embodiment 6: Study on the hygroscopicity

About 10 to 15 mg of the sample was subjected to DVS detection, and the test results was as shown in Table 8

**Table 8: Table of hygroscopic information**

| **Compound** | **Hygroscopic weight gain at 25/80% RH** |
|---|---|
| **Crystal form A of the compound represented by formula (I)** | 0.2801 % |
| **Crystal form C of the compound represented by formula (II)** | 0.999% |

Conclusion: The crystal form A of the compound represented by formula (I) and the crystal form C of the compound represented by formula (II) are slightly hygroscopic.

### Embodiment 7: Enzymatic activity test of the compound represented by formula (I)

### Reagents and consumables:

Reaction buffer: 20 mM Hepes (pH 7.5), 10 mM MgCl₂, 1 mM EGTA, 0.02% Brij35, 0.02 mg/mL BSA (bovine serum albumin), 0.1 mM Na₃VO₄, 2 mM DTT (dithiothreitol), 1% DMSO and corresponding cofactors

### Preparation of the compound:

Test compound and reference compound were diluted with 100% DMSO to 0.33 µM, then fully automated microplate pretreatment system ECHO was used for a 3-fold dilution with 10 concentration gradients.

### Reaction operation:

1) The substrate was dissolved in freshly prepared buffer,
2) The required cofactors was added to the buffer,
3) The enzyme was added to the above solution and the mixture was mix well,
4) The test sample solution was added and incubated for 20 min at room temperature,
5) ³³p-ATP was added to the reaction mixture and then incubated at room temperature for 2 hours,
6) Radiation signal was detected,
7) The results were analyzed with GraphPad prism software.

**Experimental result:** As shown in Table 9.

**Table 9: The IC₅₀ value of the compound represented by formula (I) on the inhibition of kinase activity**

| **Test compound** | **AXL IC₅₀ (nM)** | **c-MET IC₅₀ (nM)** |
|---|---|---|
| Crystal form A of the compound represented by formula (I) | 4.41 | 2.01 |

The experimental result shows that the compound represented by formula (I) has strong inhibitory activity on c-MET and AXL enzyme.

### Embodiment 8: Cell proliferation inhibition experiment of the compound represented by formula (I)

### Reagents and consumables:

1) Cell culture: DMEM medium, fetal bovine serum, DPBS
2) Cell line: MKN45 gastric cancer cell line
3) Detection reagent: live cell detection kit CellTiter-Glo
4) Other major consumables and reagents: compound dilution plate, intermediate plate, test plate, DMSO

### Experimental principle:

The content of ATP directly reflects the number of cells and their status, and the number of live cells can be detected by quantitative determination of ATP. The Live Cell Assay Kit contains fluorogenic luciferase and its substrate. Through the involvement of ATP, luciferase can catalyze the substrate and emit a stable optical signal, and the content of ATP in the cell can be measured by detecting the intensity of the signal. The light signal is directly proportional to the amount of ATP in the cell, and ATP is positively related to the number of living cells, so that the cell proliferation can be detected. The test plate was analyzed by Envision of PE company.

### Experimental method:

### 1. Preparation of the cell plates

MKN45 cells were seeded separately into 384-well plates with each of the well containing 200 cells. The cell plates were placed and incubated in a carbon dioxide incubator overnight.

### 2. Preparation of the compound

Echo (automatic microplate pretreatment system) was used for 5-fold dilution and 9 concentrations were prepared, double duplicate wells assay was set up.

### 3. Treatment of cells with the compound

The compound was transferred to the cell plates at a starting concentration of 10 µM. The cell plates were incubated in a carbon dioxide incubator for 3 days.

### 4. Detection

The Promegaer CellTiter-Glo reagent was added to the cell plates and the plates were incubated at room temperature for 10 minutes until the luminescence signal was stable. Reading was performed with a PerkinElmer Envision multi-label analyzer.

**Experimental result:** As shown in Table 10.

**Table 10: The IC₅₀ value of the Crystal form A of the compound represented by formula (I) on cell proliferation inhibition**

| **Cell name** | **IC₅₀ (nM)** |
|---|---|
| **MKN45 cells** | 7.64 |

The result of the experiment shows that the crystal form A of the compound represented by formula (I) has good inhibitory activity on MKN45 cell.

### Embodiment 9: In vivo pharmacodynamic studies of the compound represented by formula (I)

### Cell culture:

Human gastric cancer HS 746T cells were cultured in a single layer *in vitro.* The culturing condition was DMEM medium supplemented with 10% fetal bovine serum, 100U/mL penicillin and 100U/mL streptomycin in 37 °C, 5% CO₂ incubator. Digestion and passage treatment with trypsin-EDTA was carried out twice a week. When the cell saturation was 80%-90% and the number reached the required level, the cells were collected, counted and seeded.

### Animal:

BALB/c nude mice, male. 6-8 weeks old, weighting 18-22 g.

### Tumor inoculation:

0.2 mL (2×10⁶, cells: Matrigel=1:1) HS 746T cells were inoculated subcutaneously on the right back of each mouse. The drug was administered in groups when the average tumor volume reached approximately 100-150 mm³.

Experimental index: The experimental index was whether the tumor growth was inhibited, delayed or cured. The diameters of the tumor were measured twice a week using a vernier caliper. The formula for calculating the tumor volume is V = 0.5a× b², a and b represent the long and short diameters of the tumor respectively. The antitumor effect (TGI) of the compound was evaluated by T-C (days) and T/C (%).

Experimental results: As shown in Table 11.

**Table 11: Evaluation of anti-tumor efficacy of test drug on human Hs746t gastric cancer cell xenograft tumor model**

| (Calculated based on the tumor volume on the 21st day after administration) | | | | |
|---|---|---|---|---|
| Group | Tumor volume (mm³)^{a} | T/C (%) | TGI (%) | *P* vaule^{b} |
| | (20th day) | | | |
| Blank | 2537±425 | - | - | - |
| BMS777607 | 1872±355 | 27.61% | 73.58 | <0.001 |
| LY2801653 | 88±13 | 101.62% | 3.45 | |
| Compound represented by formula (I) | 4±2 | 104.82% | 0.15 | <0.001 |

| | | | | |
|---|---|---|---|---|
| Note: a. average value±SEM; b. p value was calculated based on the tumor volume. | | | | |

Conclusion: The compound represented by formula (I) shows better tumor inhibitory effect than BMS777607 and LY2801653 in the pharmacodynamic experiment on Hs746t gastric cancer cell xenograft tumor model.

## Claims

1. A crystal form A of a compound represented by formula (I), the X-ray powder diffraction pattern thereof has characteristic diffraction peaks at the following 2θ angles: 9.37° ± 0.20°, 17.17° ± 0.20°, and 18.89°±0.20°.

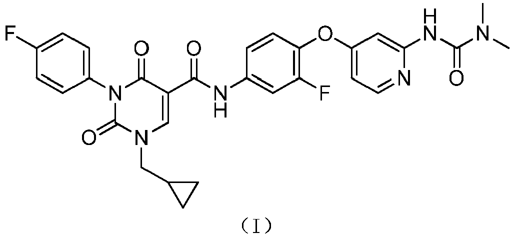

2. The crystal form A as defined in claim 1, the X-ray powder diffraction pattern thereof has characteristic diffraction peaks at the following 2θ angles: 9.37°±0.20°, 10.37±020°, 12.92±020°, 17.17±0.20°, 18.89±0.20°, 19.82±0.20°, 22.09±0.20° and 24.48±0.20.

3. The crystal form A as defined in claim 2, the X-ray powder diffraction pattern thereof has characteristic diffraction peaks at the following 2θ angles: 8.10°, 9.37°, 10.37°, 10.92°, 12.92°, 14.11°, 14.67°, 15.21°, 15.85°, 16.21°, 16.66°, 17.17°, 17.64°, 18.89°, 19.18°, 19.82°, 20.74°, 21.30°, 22.09°, 22.91°, 23.90°, 24.48°, 25.56°, 25.92°, 26.29°, 27.04°, 27.39°, 28.32°, 29.27°, 29.86°, 30.57°, 31.34°, 32.16°, 32.62°, 33.27°, 33.79°, 34.45°, 34.75°, 36.80° and 39.33°.

4. The crystal form A as defined in any one of claims 1 to 3, the differential scanning calorimetry curve thereof has an endothermic peak with onset at 206.05 °C±3 °C.

5. The crystal form A as defined in claim 4, the differential scanning calorimetry curve pattern thereof is as shown in Fig. 2.

6. The crystal form A as defined in any one of claims 1 to 3, the thermogravimetric analysis curve thereof has a weight loss of 0.07730% occurred at 158.11°C±3°C, and a weight loss of 1.0628% occurred at 203.86°C±3°C.

7. The crystal form A as defined in claim 6, the thermogravimetric analysis curve pattern thereof is as shown in Fig. 3.

8. A crystal form B of a compound represented by formula (I), the X-ray powder diffraction pattern thereof has characteristic diffraction peaks at the following 2θ angles: 9.19 ± 0.20°, 12.34 ± 0.20° and 16.45 ± 0.20°.

9. The crystal form B as defined in claim 8, the X-ray powder diffraction pattern thereof has characteristic diffraction peaks at the following 2θ angles: 9.19±0.20°, 12.34±020°, 16.45±020°, 16.88±0.20°, 18.95±0.20°, 21.34±0.20°, 22.39±0.20° and 24.34±0.20°.

10. The crystal form B as defined in claim 9, the X-ray powder diffraction pattern thereof has characteristic diffraction peaks at the following 2θ angles: 6.35°, 9.19°, 10.00°, 12.34°, 12.74°, 13.57°, 16.55°, 16.88°, 17.40°, 17.80°, 18.28°, 18.95°, 19.60°, 20.19°, 21.34°, 21.69°, 22.39°, 23.33°, 23.68°, 24.34°, 24.73°, 25.56°, 26.35°, 26.94°, 27.69°, 28.36°, 29.03°, 29.35°, 30.06°, 30.55°, 31.12°, 33.19°, 33.86°, 34.10°, 36.01° and 36.66°.

11. The crystal form B as defined in any one of claims 8 to 10, the differential scanning calorimetry curve thereof has endothermic peaks with onset at 136.23°C±3°C and 206.26±3 °C.

12. The crystal form B as defined in claim 11, the differential scanning calorimetry curve pattern thereof is as shown in Fig. 5.

13. The crystal form B as defined in any one of claims 8 to 10, the thermogravimetric analysis curve thereof has a weight loss of 7.912% occurred at 136.32°C±3°C, and a weight loss of 9.993% occurred at 198.78°C±3°C.

14. The crystal form B as defined in claim 13, the thermogravimetric analysis curve pattern thereof is as shown in Fig. 6.

15. A compound represented by formula (II).

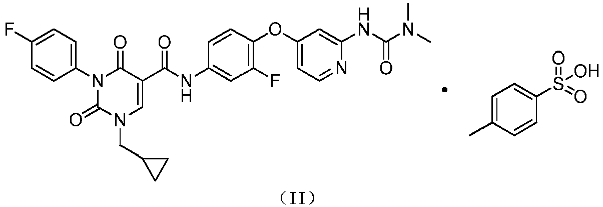

16. A crystal form C of a compound represented by formula (II), the X-ray powder diffraction pattern thereof has characteristic diffraction peaks at the following 2θ angles: 4.22 ± 0.20°, 14.91 ± 0.20° and 20.75 ± 0.20°.

17. The crystal form C as defined in claim 16, the X-ray powder diffraction pattern thereof has characteristic diffraction peaks at the following 2θ angles: 4.22±0.20°, 10.23±020°, 14.34±020°, 14.91±0.20°, 19.27±0.20°, 19.94±0.20°, 20.75±0.20°, 23.51±0.20°, 28.38±0.20°, 29.03±0.20° and 29.50±0.20°.

18. The crystal form C as defined in claim 17, the X-ray powder diffraction pattern thereof has characteristic diffraction peaks at the following 2θ angles: 4.22°, 7.18°, 8.26°, 10.23°, 13.47°, 14.34°, 14.91°, 15.68°, 16.06°, 16.48°, 17.07°, 17.67°, 18.12°, 18.65°, 19.27°, 19.94°, 20.35°, 20.75°, 21.55°, 22.23°, 22.48°, 23.51°, 24.73°, 25.34°, 26.07°, 26.35°, 26.94°, 27.25°, 27.63°, 28.38°, 29.03°, 29.50°, 29.96°, 30.57°, 31.24°, 32.03°, 32.91°, 33.59°, 34.32°, 34.94°, 35.79°, 37.69° and 38.28°.

19. The crystal form C as defined in any one of claims 16 to 18, the differential scanning calorimetry curve thereof has an endothermic peak with onset at 220.74 °C±3 °C.

20. The crystal form C as defined in claim 19, the differential scanning calorimetry curve pattern thereof is as shown in Fig. 8.

21. The crystal form C as defined in any one of claims 16 to 18, the thermogravimetric analysis pattern thereof has a weight loss of 0.004784% occurred at 159.80°C±3 °C.

22. The crystal form C as defined in claim 21, the thermogravimetric analysis curve pattern thereof is as shown in Fig. 9.

23. A compound represented by formula (III).

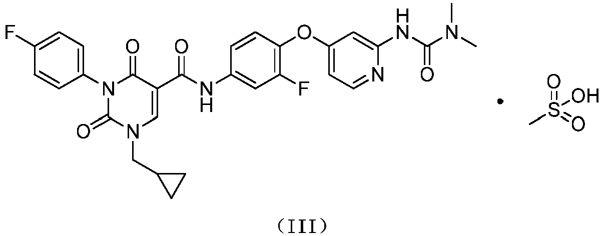

24. A crystal form D of a compound represented by formula (III), the X-ray powder diffraction pattern thereof has characteristic diffraction peaks at the following 2θ angles: 7.49 ± 0.20°, 9.64 ± 0.20°and 19.23 ± 0.20°.

25. The crystal form D as defined in claim 24, the X-ray powder diffraction pattern thereof has characteristic diffraction peaks at the following 2θ angles: 7.49±0.20°, 9.64±0.20°, 18.75±0.20°, 19.23±0.20°, 20.93±0.20°, 21.55±0.20° and 22.17±0.20°.

26. The crystal form D as defined in claim 25, the X-ray powder diffraction pattern thereof has characteristic diffraction peaks at the following 2θ angles: 7.49°, 7.89°, 8.50°, 9.17°, 9.64°, 11.20°, 11.67°, 12.28°, 14.93°, 15.40°, 17.35°, 18.75°, 19.23°, 20.93°, 21.55°, 22.17°, 23.31°, 24.12°, 24.88°, 25.58°, 26.53°, 27.53° and 31.10°.

27. The crystal form D as defined in any one of claims 24 to 26, the differential scanning calorimetry curve thereof has an endothermic peak with onset at 223.59°C±3 °C.

28. The crystal form D as defined in claim 27, the differential scanning calorimetry curve pattern thereof is as shown in Fig. 11.

29. The crystal form D as defined in any one of claims 24 to 26, the thermogravimetric analysis curve thereof has a weight loss of 0.3850% occurred at 150.12°C±3 °C.

30. The crystal form D as defined in claim 29, the thermogravimetric analysis curve pattern thereof is as shown in Fig. 12.

31. A compound represented by formula (IV).

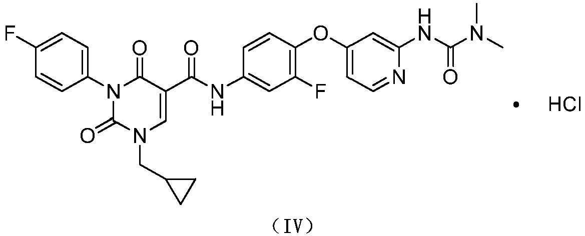

32. A crystal form E of a compound represented by formula(IV), the X-ray powder diffraction pattern thereof has characteristic diffraction peaks at the following 2θ angles: 6.94 ± 0.20°, 10.00 ± 0.20° and 11.73±0.20°.

33. The crystal form E as defined in claim 32, the X-ray powder diffraction pattern thereof has characteristic diffraction peaks at the following 2θ angles: 5.85±0.20°, 6.94±0.20°, 10.00±0.20°, 11.73±0.20°, 15.82±0.20°, 17.10±0.20°, 20.39±0.20° and 23.74±0.20°.

34. The crystal form E as defined in claim 33, 5.85°, 6.94°, 10.00°, 11.73°, 13.83°, 14.41°, 15.82°, 16.38°, 17.10°, 17.47°, 18.06°, 18.95°, 20.00°, 20.39°, 20.88°, 22.25°, 23.74°, 24.91°, 25.48°, 26.39°, 27.57°, 29.86°, 30.49°, 32.62°, 35.79° and 37.14°.

35. The crystal form E as defined in any one of claims 32 to 34, the differential scanning calorimetry curve thereof has exothermic peaks with onset at 67.18°C±3°C and 203.17°C±3°C, and has endothermic peaks with onset at 181.72°C±3°C and 201.40°C±3 °C.

36. The crystal form E as defined in claim 35, the differential scanning calorimetry curve pattern thereof is as shown in Fig. 14.

37. The crystal form E as defined in any one of claims 32 to 34, the thermogravimetric analysis curve thereof has a weight loss of 0.5018% occurred at 52.80°C±3°C, a weight loss of 4.4958% occurred at 173.60°C±3°C, and a weight loss of 5.8808% occurred at 210.40°C±3°C.

38. The crystal form E as defined in claim 37, the thermogravimetric analysis curve pattern thereof is as shown in Fig. 15.

39. A use of the compound or the crystal form as defined in any one of claims 1 to 38 in the manufacture of a medicament for treating cancer.
